# EUROPEAN PATENT SPECIFICATION

(11) **EP 2 052 037 B1**
(45) Date of publication and mention of the grant of the patent: **10.07.2013**
(21) Application number: 06823761.9
(22) Date of filing: 28.11.2006
(51) Int. Cl.: C09C 1/40, C09C 1/64, A61K 8/00

(54) **FLAKE PIGMENT AND PREPARATION METHOD THEREOF AND COSMETIC USING THE SAME**
SCHUPPENPIGMENT, HERSTELLUNGSVERFAHREN DAFÜR SOWIE DAVON GEBRAUCH MACHENDES KOSMETIKUM
PIGMENT EN PAILLETTES ET SON PROCÉDÉ DE PRÉPARATION, COMPOSITION COSMÉTIQUE RENFERMANT CE DERNIER

(30) Priority: 14.08.2006 KR 20060076648
(43) Date of publication of application: 29.04.2009
(73) Proprietor: CQV CO., Ltd., Chungcheongbuk-do 365-841 (KR)
(72) Inventor: KANG, Kwang Choong, Cheongju-si, Chungcheongbuk-do 360-100 (KR); JO, Sung Yun, Cheongwon-gun, Chungcheongbuk-do 363-781 (KR); LIM, Kwang Su, Cheongju-si, Chungcheongbuk-do 360-100 (KR); CHANG, Kil Wan, Cheongju-si, Chungcheongbuk-do 360-100 (KR)
(74) Representative: Grünecker, Kinkeldey, Stockmair & Schwanhäusser
(86) International application number: PCT/KR2006/005053
(87) International publication number: WO 2008/020665

(56) References cited:
- EP-A1- 1 213 330
- EP-A2- 0 763 573
- GB-A- 1 533 430
- GB-A- 1 533 430
- US-A- 5 702 519
- DATABASE WPI Week 199608, Derwent Publications Ltd., London, GB; AN 1996-075158, XP008102846 MAEDA M. ET AL & JP 07 331110 A (AGENCY OF IND SCIENCE & TECHNOLOGY ET AL.) 19 December 1995
- DATABASE WPI Week 199954, Derwent Publications Ltd., London, GB; AN 1999-622720, XP008102845 MINAMI K. ET AL & JP 11 152423 A (TOYO ALUMINIUM KK ET AL.) 08 June 1999

## Description

### Technical Field

The present invention relates to a flake pigment, a preparation method thereof and a cosmetic composition comprising the same, and particularly to a flake pigment prepared by coating a metal oxide on the surface of a flake synthetic alumina having predetermined ranges of an aspect ratio and an average particle diameter, wherein the pigment shows interference colors while reducing the glossiness, thereby enabling cosmetics containing the pigments to be superior in natural transparency and adhesive and slipping properties as well as in color expression and a mat feel due to a low glossiness, along with a preparation method thereof and a cosmetic composition comprising the same.

### Background Art

A pigment used in a cosmetic composition is preferred to show a good skin feel (such as slipping and adhesive properties), an appropriate hiding effect (such as semitransparency or natural skin color) and an appropriate glossiness by suppressing excessive glossiness caused by reflected light.

Among various pigments, a flake substrate shows a relatively good slipping property and hiding power ( a skin covering effect), and has thus been developed as an extender pigment for a cosmetic raw material of, for example, compact, cake, foundation and cream.

Layered clay mineral such as talc , mica, caoline and s ericite are well known as an example of an ingredient used in a flake substrate. However, talc, mica and s ericite have a drawback of a low adhesive property, and caoline and an precipitated calcium carbonate used for increasing the adhesive property are also limited in use due to a low spreading property.

Korean patent publication Nos. 2003-0063218 and 2001-0030024 and Japanese patent publication No. 1999-199441 have suggested the use of pearl pigments for manufacturing an extender pigment for cosmetics having a good skin-adhesive property, an improved soft-focusing effect and an appropriate hiding power.

However, Korean patent publication No. 2003-0063218, which relates to a preparation method comprising the step of attaching finely-granulated (coagulated) TiO₂ on mica particles through the change in pH, has drawbacks of a rough usage feel due to a problem in dispersion, a low skin spreadability and a poor expression of interference colors, although it may be able to maintain whiteness when used as a raw material for cosmetics.

Korean patent publication No. 2001-0030024 attempted to accomplish the aforementioned object by coating fine granules of calcium carbonate on a flake substrate, resulting in the same drawbacks due to high degree of coagulation. Moreover, the excessive use of white pigments for increasing the use effect aggravates the whitening effect on skin despite an increase in an hiding power.

GB 1533430 relates to rutile-containing lustrous pigments and EP 1213330 discloses silver lustrous pigments coated with layers of TiO2. Japanese patent publication No. 1999-199441 discloses a cosmetic powder comprising flake mica coated with rutile-type TiO₂ micro-granules. However, the Japanese patent publication has a problem of excessive glossiness due to the metallic glossiness of TiO₂.

Besides, Korean patent publication No. 1996-0029421 suggests that the aspect ratio be limited to higher than 60 for good interference effect when using mica.

EP 0763573 is concerned with the production of a flaky aluminum oxide and pearlescent pigments. Meanwhile, as a way to use synthetic alumina particles, i.e., one kind of flake substrate, Korean patent publication Nos. 2002-0067404 and 1997-0015682 discloses flake synthetic alumina particles having an average particle diameter of 0.5-25 µm and an aspect ratio of 50-2000. However, when an aspect ratio is higher than 200, the structure may induce an optical glossiness, and cause lack of a mat feel due to glossiness resulting from high aspect ration when applied to cosmetics.

Japanese patent publication No. 2003-192338 aims to provide a f lake synthetic alumina that is superior in a mat feel and skin spreading property and has improved skin adhesive property and hiding power, while maintaining glossiness. However, the aforementioned Japanese patent publication fails to obtain a mat feel due to excessive glossiness caused by 20 µm or higher particle size and high aspect ratio, and also fails to accomplish interference colors by using only 1-90 wt% of flake alumina particles.

To solve the aforementioned problems, the present inventors have exerted extensive researches to control the glossiness and prepare flake synthetic alumina particles that enable the expression of interference colors . As a result, the present invention has been completed on a basis of the finding that a flake pigment, which is prepared by coating a metal oxide on the surface of the flake synthetic aluminum particle having an aspect ratio of 10-50, an average particle diameter of 2-20 µm, enables a low glossiness and the expression of interference colors at the same time without using additional additives.

Furthermore, the present invention is also based on the fact that cosmetics comprising the aforementioned flake pigment is superior in transparency on the skin and adhesive and slipping property, and enables a mat feel and color expression due to low glossiness.

Therefore, the present invention aims to provide a flake pigment that enables low glossiness and color expression through the interference colors, along with a preparation method thereof and cosmetic comprising the same.

### Disclosure

The present invention relates to a flake pigment, wherein a metal oxide is coated on the surface of flake synthetic alumina particles having an aspect ratio of 10-50 and an average particle diameter of 2-20 µm, and luminance (L) is in the range of 50-80 and values of a and b are in the ranges of between -15 and 15 and between -25 and 20, respectively.

Hereunder is provided a more detailed description of the present invention.

The present invention relates to a flake pigment prepared by coating a metal oxide on the surface of flake synthetic alumina having predetermined ranges of aspect ratio and average particle diameter. The flake pigment of the present invention enables the expression of the interference colors while maintaining low glossiness. Thus, the cosmetics containing the pigment of the present invention shows natural transparency and adhesive and slipping property and enables colors expression and a mat feel caused by low glossiness, thereby being useful in foundation , lipstick, eye shadow, mascara and other make-up cosmetics, milk lotions and creams.

In general, a flake pigment is used to provide high glossiness and high chroma, i.e., to increase the glossiness along with an effector such as color. However, when the glossiness is extremely high, cosmetics decrease in a mat feel and other functions , thereby causing limitation in selection of pigments. The optical or other properties of a flake pigment depend on an aspect ratio, which is defined as a ratio of a diameter to a thickness of a pigment particle. When an aspect ratio is 100 or higher, preferably 200 or higher, it is may be used as a flake substrate that may serve as a pigment showing an increased glossiness as well as expressing a quality interference color. When an aspect ratio is 20 or less, preferably 10 or less, the optical property is aggravated and usage feel is lowered due to the decrease in adhesive and slipping property as a pigment.

Therefore, a flake pigment of the present invention shows low glossiness as well as interference color by controlling glossiness, which is an effect accomplished by lowering the aspect ratio as compared to the flake synthetic alumina that is conventionally used as a cosmetic pigment ingredient and selecting an average diameter of 2-20 µm.

Hereunder is provided a detailed description of a method for preparing a flake pigment according to the present invention.

The pH is adjusted to 1-3 by adding an acid to an aqueous solution of flake synthetic alumina particles. The flake synthetic alumina has preferably an aspect ratio of 10-50, an average particle diameter of 2-20 µm. If the aspect ratio is lower than 10, the flake synthetic alumina may not be used as a pigment for interference colors due to the difficulty in expression of the interference colors. If the aspect ratio is higher than 50, a mat feel may not be obtained due to the high gloss of particles. If the average particle diameter is less than 2 µm, a metal oxide layer may not be formed due to the coagulation of particles . An average particle diameter above 20 µm may cause a spectacular glossiness of particles like in a metal.

Generally, a low aspect ratio causes difficulty in expression of interference colors and deteriorates the decrease in adhesive and slipping property of particles, thereby lowering usage feed and keeping the particles from being used as a cosmetic pigment. However, the present invention relates to selection of an appropriate range of aspect ratio.

Any conventional organic or inorganic acid, such as acetic acid, butylic acid, nitric acid, chloric acid and sulfuric acid, may be used as the aforementioned acid. The acid is preferably adjusted to be in the range of pH 1-3 for an appropriate rate of hydrolysis of metal to coat. If a pH is lower than 1, it would be difficult to efficiently coat a metal oxide. If the pH is higher than 3, quality interference colors may not be obtained because coagulation occurs due to a rapid hydrolysis.

Then, an aqueous solution of flake synthetic alumina particle s coated with a hydrated metal oxide layer is prepared by adding a metal chloride solution and an alkaline aqueous solution in the acid-added aqueous solution of the flake synthetic alumina particles at the same time followed by reflux.

The metal chloride is conventionally used to apply a coating onto the surface of pigments, thereby expressing metal glossiness and interference colors. The examples of the metal chloride include without limitation SnCl₄, TiOCl₂, ZrOCl₂, SiCl₄, AlCl₃, MgCl₂, MnCl₂ and a mixture thereof. It is preferred to form a plurality of coating layers by using the metal chloride, for example, a coating layer of SnCl₄ and another coating layer of TiOCl₂ on the SnCl₄ coating layer. The aforementioned multi-coating layer has advantages of an enhanced hiding power and quality interference colors .

The metal chloride solution is used in such an amount that 20-55 wt% of metal oxide is finally formed on the flake alumina particles. If the amount is lower than 20 wt%, a metal oxide layer may be too thin and sufficient interference colors may not be obtained. If the amount is higher than 55 wt%, it may cause the light scattering and the whitening due to the formation of an excessively thick TiO₂ layer, thereby making it difficult to express quality colors.

To form a hydrated metal oxide layer, an alkaline aqueous solution, such as sodium hydroxide, potassium hydroxide , ammonia and sodium carbonate may be used along with the metal chloride solution.

The alkaline aqueous solution is preferred to be used in a diluted concentration of 10-15% with a metal chloride solution so that a good coating layer may be formed on the flake alumina and that a constant pH may be maintained.

The alkaline aqueous solution is used preferably in a weight ratio of 1-1.2 relative to the metal chloride so that 20-55 wt% of a metal oxide may be formed on the relative to the flake aluminum particle. If the weight ratio of the alkaline aqueous solution is lower than 1, it may cause a delayed formation of the metal oxide layer. If the weight ratio is higher than 1.2, a quality metal oxide layer may not be obtained due to coagulation resulted from a rapid hydrolysis reaction.

Then, pigments are prepared by filtering, washing and drying the aqueous solution of the flake synthetic alumina particles coated with the hydrated metal oxide layer, followed by carcination .

The washing may be performed by using deionized water and repeating the same procedure 2-5 times. The drying may be performed using a drying oven at 90-120 °C. If a drying temperature is lower than 90 ° C, it may take too long for drying. If a drying temperature is higher than 120 ° C, quality interference colors may not be obtained.

The carcination may be performed at 700-1,000 ° C for 30-60 minutes. If a carcination temperature is lower than 700 ° C, it may become difficult to crystallize TiO₂. If a carcination temperature is higher than 1,000 ° C, it may raise problems of the coagulation of particles and the cracks of the coated metal oxide layer due to the high carcination temperature.

Thus prepared flake pigments of the present invention is superior in expressing low glossiness along with quality interference colors without using other additives .

In another preferred embodiment, the present invention also relates to cosmetics comprising the aforementioned flake pigment. The flake pigment may be contained in the cosmetics in the conventional dosage.

Besides pigments, cosmetics further comprises in general used additives such as higher aliphatic alcohol, higher fatty acid, ester oil, paraffin oil, wax or other oily ingredients, ethyl alcohol, propylene glycol, sorbitol, glucose or other alcohols, mucopolysaccharide, collagen, lactate or other moisturizers, various surfactants, a thickener, an anti-oxidant, pH buffer, a preservative and an aromatic.

Cosmetics comprising the flake pigments of the present invention show a superior usage feel without excessive glossiness and coagulation, along with quality interference color effect with superior hiding powder or a mat effect.

### Best Mode

The present invention is described more specifically by the following Examples. Examples herein are meant only to illustrate the present invention, but they should not be construed as limiting the scope of the claimed invention.

**Example 1**

In 2 L of deionized water were suspended 100 g of synthetic alpha-alumina particles having 2-20 µm of particle size, 10-50 of aspect ratio and 1.76-1.77 of diffraction ratio, and the temperature was elevated to about 80 ° C. 5% chloric acid was added to lower the pH to about 2.0 followed by reflux for more than 30 minutes. 40% TiOCl₂ and 15% diluted sodium hydroxide solution were added together to adjust the pH to 2.0. As the amount of TiOCl₂ added increases, the solution gradually took on silver (120 mL), gold (180 mL), red (215 mL), blue (270 mL) and green (320 mL). When a desired color was obtained, the addition of TiOCl₂ was stopped and reflux was conducted for more than 10 minutes. The acidic solution was filtered, washed three times with deionized water, dried at 110 ° C and calcined at 850 ° C for 30 minutes to obtain a flake pigment.

Analysis of the calcined pigment according to X-ray diffraction method shows that Anatase-structured titanium dioxide layer was formed on the surface of the synthetic alpha-alumina.

**Example 2**

In 2 L of deionized water were suspended 100 g of synthetic alpha-alumina particles having particle size of 2-20 µm, aspect ratio of 10-50 and diffraction ratio of 1.76-1.77, and the temperature was elevated to about 80 ° C. 5% chloric acid was added to lower the pH to about 1.5 followed by reflux for more than 30 minutes. 30 mL of 5% SnCl₄·5H₂O solution and 15% diluted sodium hydroxide solution were added together while adjusting the pH to 2.0, and reflux was conducted for 30 minutes. As the amount of SnCl₄ added increased, the solution gradually took on silver (140 mL), gold (210 mL), red (250 mL), blue (315 mL) and green (375 mL). When a desired color was obtained, the addition of SnCl₄ was stopped and reflux was conducte d for more than 10 minutes. A necessary amount of the acidic solution was filtered, washed, dried and calcined as described in Example 1 to obtain a flake pigment.

Analysis of the calcined pigment according to X-ray diffraction method shows that Rutile-structured titanium dioxide layer was formed on the surface of the synthetic alpha-alumina.

**Comparative Example 1: Korean Patent Publication No.** 1997-15682

The same procedure was followed as in Example 2 except by using synthetic alpha-alumina having particle size of 5-60 µm and aspect ratio of about 100, thus obtaining pigments of silver, gold , red, blue and green colors, respectively.

Analysis of the obtained pigment according to X-ray diffraction method shows that Rutile-structured titanium dioxide layer was formed on the surface of the synthetic alpha-alumina.

**Experimental Example 1**

Pigments prepared in Example 2 and Comparative Example 1 were measured of their glossi ness, and the results are provided in Table 1 .

[Measurement of glossiness]

0.3 g of pearl pigment was sufficiently dispersed in 5 g of nitro cellulose with a viscosity of 1,200 cps. The dispersed solution was moved to a draw-down card and uniformly drawn down by using Doctor Applicators (blade, Gape 100 µm), followed by drying at room temperature. The glossiness was measured at 60 ° with a gloss sensor(Nippon Denshoku Co., Ltd. PG-1M).

**Table 1**

| Run | Silver | Gold | Red | Blue | Green |
|---|---|---|---|---|---|
| Ex. 2 | 27.9 | 25.7 | 22 | 21.6 | 22.3 |
| Comp. Ex. 1 | 52.4 | 48.7 | 49.2 | 34.4 | 39.6 |

As shown in Table 1, pigments of Example 2 have lower glossiness than that of Comparative Example 1.

**Experimental Example 2**

Color difference was measured for the pigments of Example 2 and a lumina, s ericite and talc , which are commonly used as extender pigment, and the results are presented in Table 2. The relative values of color difference (da*, db*, dE*) were obtained in the other seven kinds of pigments on a basis of color difference of alumina.

[Measurement of color difference using a colorimeter]

Color difference was measured at 25 ° with as pectrophotometer of CM-512M3 (KONICA MINOLTA SENSING, INC.) by using a draw-down card, which was drawn down as described in Experimental Example 1.

**Table 2**

| Run | | L* | a* | b* | da* | db* | dE* |
|---|---|---|---|---|---|---|---|
| Example 2 | Silver | 76.79 | -3.35 | -4.27 | -3.27 | -3.33 | 36.28 |
| | Gold | 67.81 | -0.35 | 19.31 | -0.27 | 20.24 | 33.33 |
| | Red | 52.48 | 14.28 | -8.27 | 14.46 | -7.33 | 19.49 |
| | Blue | 52.24 | -3.29 | -25.85 | -3.21 | -24.92 | 26.69 |
| | Green | 64.54 | -15.04 | 3.33 | -14.96 | 4.26 | 28.08 |
| Alumina | | 40.79 | -0.08 | -0.96 | | | |
| Sericite | | 32.67 | 0.14 | -1.37 | 0.22 | -0.43 | 8.13 |
| Talc | | 36.12 | 0.06 | -1.57 | 0.14 | -0.63 | 4.71 |
| da*, db* and dE*: Relative color difference values | | | | | | | |
| L*: Luminance | | | | | | | |
| a*, b*: Color coordinates | | | | | | | |

As shown in Table 2, alumina , s ericite and talc were low in luminance and color coordinate, leading to difficulty in realizing colors (white powder), and the pigments of Example 2 have their unique interference colors.

**Example 3**

Cosmetics of a compact, a foundation and powder were prepared by using pigments prepared in Example 2 and Comparative Example 1, and the contents are presented in Tables 3-5.

**Table 3**

| Run | Compact containing Ex. 2 (wt%) | Compact containing Comp. Ex. 2 (wt%) |
|---|---|---|
| Ex. 2 | 15.0 | - |
| Comp. Ex. 2 | - | 15.0 |
| Mica | 5.0 | 5.0 |
| Silica | 8.0 | 8.0 |
| Titanium dioxide | 3.0 | 3.0 |
| Iron oxide | 0.5 | 0.5 |
| Dimethicone | 7.0 | 7.0 |
| Perfume | 0.1 | 0.1 |
| Talc | 61.4 | 61.4 |

**Table 4**

| Run | Foundation containing Ex. 2 (wt%) | Foundation containing Comp. Ex. 2 (wt%) |
|---|---|---|
| Ex. 2 | 10 | - |
| Comp. Ex. 2 | - | 10 |
| Seresin | 1.0 | 1.0 |
| Candelilla Wax | 2.0 | 2.0 |
| Cyclomethicon | 7.0 | 7.0 |
| Mica | 5.0 | 5.0 |
| Paraffin oil | 40.0 | 40.0 |
| Perfume | 0.2 | 0.2 |
| Distilled water | 34.8 | 34.8 |

**Table 5**

| Run | Power containing Ex. 2 (wt%) | Power containing Comp. Ex. 2 (wt%) |
|---|---|---|
| Ex. 2 | 35.0 | - |
| Comp. Ex. 2 | - | 35.0 |
| Iron oxide for pigment | 0.3 | 0.3 |
| Titanium oxide | 1.0 | 1.0 |
| Silicone resin powder | 8.5 | 8.5 |
| Dimethyl polysiloxane | 10 | 10 |
| Talc | 45.1 | 45.1 |
| Perfume | 0.1 | 0.1 |

The resulting cosmetics were subject to the measurement of the properties such as spreadability , hiding power, interference color, mat feel and excessive glossiness , and the results are presented in Table 6.

[Method for the measurement of properties]

For the measurement of spreadability , hiding power , interference color , mat feel and excessive glossiness, the cosmetics shown in Table 3-5 were applied to the facial skins of a female panel in their 20-40 ' s. Sensory evaluation was performed and the results are presented in Table 6.

The values were expressed based on the following standards: x: bad, Δ: average, ○: good, ⊚: excellent, ⊙: super excellent

**Table 6**

| Kinds of cosmetics | Pigment | Spreadability | Hiding power | Interference colors | Mat feel | Excessive glossiness |
|---|---|---|---|---|---|---|
| Compact | Ex. 2 | □ | □ | ⊚ | □ | × |
| | Comp. Ex.1 | × | × | ⊚ | × | □ |
| Foundation | Ex. 2 | □ | □ | ⊚ | ⊚ | × |
| | Comp. Ex. 1 | Δ | × | ⊚ | × | □ |
| Powder | Ex. 2 | □ | □ | ⊚ | □ | × |
| | Comp. Ex. 1 | × | × | ⊚ | × | □ |

As shown in Table 6, the cosmetics prepared using the pigment of Example 2 are superior in non-glossiness , spreadability , hiding power, transparency and interference colors. Therefore, it was confirmed that the cosmetics of the present invention is superior in color expression due to low gloss and interference colors, while retaining equivalent or superior functions to those of the commonly used cosmetics.

### Industrial Applicability

As described above, the present invention relates to a flake pigment prepared by coating a metal oxide on the surface of flake synthetic alumina having predetermined ranges of aspect ratio and average particle diameter. The flake pigment of the present invention enables the expression of the interference colors while maintaining low glossiness. Thus, the cosmetics containing the pigment of the present invention shows natural transparency and adhesive and slipping property and enables colors expression and a mat feel caused by low glossiness, thereby being useful in foundation , lipstick, eye shadow, mascara and other make-up cosmetics, milk lotions and creams.

## Claims

1. A flake cosmetics pigment, wherein a metal oxide is coated on the surface of flake alumina particles having an aspect ratio of 10-50 and an average particle diameter of 2-20 µm, and luminance (L) which is measured at 25° with a spectrophotometer is in the range of 50-80 and values of a* and b* which are measured at 25° with a spectrophotometer are in the ranges of between -15 and 15 and between -25 and 20, respectively.

2. The flake cosmetics pigment of claim 1, wherein the pigment takes on silver color, the luminance (L) is in the range of 72-80 and the values of a* and b* are in the ranges of between - 5 and 0 and between -7 and -2, respectively.

3. The flake cosmetics pigment of claim 1, wherein the pigment takes on gold color, the luminance (L) is in the range of 65-70 and the values of a and b are in the ranges of between -3 and 2 and between 17 and 22, respectively.

4. The flake cosmetics pigment of claim 1, wherein the pigment takes on reddish color, the luminance (L) is in the range of 50-55 and the values of a* and b* are in the ranges of between 12 and 17 and between -10 and -5, respectively.

5. The flake cosmetics pigment of claim 1, wherein the pigment takes on blue color, the luminance (L) is in the range of 50-55 and the values of a* and b* are in the ranges of between - 5 and 0 and between -27 and -22, respectively.

6. The flake pigment of claim 1, wherein the pigment takes on green color, the luminance (L) is in the range of 62-67 and the values of a* and b* are in the ranges of between -17 and -12 and between 0 and -5, respectively.

7. The flake cosmetics pigment of claim 1, wherein the metal oxide is at least one selected from the group consisting of TiO₂, SnO₂, ZrO₂, MgO and MnO.

8. The flake cosmetics pigment of claim 1, wherein the metal oxide is coated on the surface of the flake synthetic alumina particles in the amount of 20-55 wt%.

## Patentansprüche

1. Ein kosmetisches Schuppenpigment bei welchem ein Metalloxid auf die Oberfläche von Schuppenteilchen aus Aluminiumoxid beschichtet wird, welche einen Formfaktor von 10-50 und einen durchschnittlichen Teilchendurchmesser von 2-20 µm haben, und eine Luminanz (L), die bei 25° mit einem Spektrometer gemessen wird, die im Bereich von 50-80 liegt, und die Werten von a* und b*, die bei 25° mit einem Spektrometer gemessen werden, die jeweils in den Bereichen von zwischen -15 und 15 und zwischen -25 und 20 liegen.

2. Das kosmetische Schuppenpigment nach Anspruch 1, wobei das Pigment eine silberne Farbe annimmt, die Luminanz (L) im Bereich von 72-80 liegt und die Werte von a* und b* jeweils in den Bereichen von -5 und 0 und zwischen -7 und 2 liegen.

3. Das kosmetische Schuppenpigment nach Anspruch 1, wobei das Pigment eine goldene Farbe annimmt, die Luminanz (L) im Bereich von 65-70 liegt und die Werte von a und b jeweils in den Bereichen von -3 und 2 und zwischen 17 und 22 liegen.

4. Das kosmetische Schuppenpigment nach Anspruch 1, wobei das Pigment eine rötliche Farbe annimmt, die Luminanz (L) im Bereich von 50-55 liegt und die Werte von a* und b* jeweils in den Bereichen von 12 und 17 und zwischen -10 und -5 liegen.

5. Das kosmetische Schuppenpigment nach Anspruch 1, wobei das Pigment eine blaue Farbe annimmt, die Luminanz (L) im Bereich von 50-55 liegt und die Werte von a* und b* jeweils in den Bereichen von -5 und 0 und zwischen -27 und -22 liegen.

6. Das kosmetische Schuppenpigment nach Anspruch 1, wobei das Pigment eine grüne Farbe annimmt, die Luminanz (L) im Bereich von 62-67 liegt und die Werte von a* und b* jeweils in den Bereichen von -17 und -12 und zwischen 0 und -5 liegen.

7. Das kosmetische Schuppenpigment nach Anspruch 1, wobei das Metalloxid mindestens eines ist, das aus der Gruppe ausgewählt wird, die aus TiO₂, SnO₂, ZrO₂, MgO und MnO besteht.

8. Das kosmetische Schuppenpigment nach Anspruch 1, wobei das Metalloxid in einer Menge von 20-55 Gewichtsprozent auf die Oberfläche der synthetischen Schuppenteilchen aus Aluminiumoxid beschichtet wird.

## Revendications

1. Pigment en paillettes pour cosmétiques, dans lequel une couche d'oxyde métallique est déposée sur la surface de particules d'alumine en paillettes ayant un rapport de forme de 10 à 50 et un diamètre particulaire moyen de 2 à 20 µm, et la luminance (L) qui est mesurée à 25° avec un spectrophotomètre se situe dans la plage de 50 à 80 et les valeurs de a* et de b* qui sont mesurées à 25° avec un spectrophotomètre se situent dans les plages respectives de -15 à 15 et de -25 à 20.

2. Pigment en paillettes pour cosmétiques selon la revendication 1, dans lequel le pigment prend une couleur argent, la luminance (L) se situe dans la plage de 72 à 80 et les valeurs de a* et de b* se situent dans les plages respectives de -5 à 0 et de -7 à -2.

3. Pigment en paillettes pour cosmétiques selon la revendication 1, dans lequel le pigment prend une couleur or, la luminance (L) se situe dans la plage de 65 à 70 et les valeurs de a et de b se situent dans les plages respectives de -3 à 2 et de 17 à 22.

4. Pigment en paillettes pour cosmétiques selon la revendication 1, dans lequel le pigment prend une couleur rougeâtre, la luminance (L) se situe dans la plage de 50 à 55 et les valeurs de a* et de b* se situent dans les plages respectives de 12 à 17 et de -10 à -5.

5. Pigment en paillettes pour cosmétiques selon la revendication 1, dans lequel le pigment prend une couleur bleue, la luminance (L) se situe dans la plage de 50 à 55 et les valeurs de a* et de b* se situent dans les plages respectives de -5 à 0 et de -27 à -22.

6. Pigment en paillettes pour cosmétiques selon la revendication 1, dans lequel le pigment prend une couleur verte, la luminance (L) se situe dans la plage de 62 à 67 et les valeurs de a* et de b* se situent dans les plages respectives de -17 à -12 et de 0 à -5.

7. Pigment en paillettes pour cosmétiques selon la revendication 1, dans lequel l'oxyde métallique est au moins un oxyde métallique sélectionné dans le groupe constitué par TiO₂, SnO₂, ZrO₂, MgO et MnO.

8. Pigment en paillettes pour cosmétiques selon la revendication 1, dans lequel la couche d'oxyde métallique est déposée sur la surface des particules d'alumine de synthèse en paillettes en quantité de 20 à 55 % en poids.
